(19) Europäisches Patentamt
European Patent Office
Office européen des brevets

(11) Publication number: **0 373 513 B1**

(12) **EUROPEAN PATENT SPECIFICATION**

(45) Date of publication of patent specification: **10.05.95**

(51) Int. Cl.[6]: **C07C 251/08**, C07C 251/12, C07F 1/08, C07F 15/06, //C23C16/18

(21) Application number: **89122601.1**

(22) Date of filing: **07.12.89**

(54) **Fluorinated beta-ketoimines and beta-ketoiminato metal complexes.**

(30) Priority: **12.12.88 US 283418**

(43) Date of publication of application:
**20.06.90 Bulletin 90/25**

(45) Publication of the grant of the patent:
**10.05.95 Bulletin 95/19**

(84) Designated Contracting States:
**DE GB NL**

(56) References cited:
**US-A- 3 356 527**
**US-A- 3 388 141**
**US-A- 3 594 216**
**US-A- 4 514 522**

**JOURNAL OF INORGANIC AND NUCLEAR CHEMISTRY, vol. 32, 1970, pages 1895-1905; M.F. RICHARDSON et al.: "Schiff bases prepared from ethylenediamine and hexafluoroacetylacetone"**

(73) Proprietor: **AIR PRODUCTS AND CHEMICALS, INC.**
**P.O. Box 538**
**Allentown, Pennsylvania 18105 (US)**

(72) Inventor: **Norman, John Anthony Thomas**
**3032 North 4th Street**
**Whitehall, PA 18052 (US)**

(74) Representative: **Kador & Partner**
**Corneliusstrasse 15**
**D-80469 München (DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

### TECHNICAL FIELD

The present invention relates to fluorinated organic ligands and volatile metal complexes formed from such ligands.

### BACKGROUND OF THE INVENTION

In the electronics industry there is a growing need for volatile sources of different metals to be used in the chemical vapor deposition (CVD) of metallic films, metal oxide films, metal silicide films, and the like. The key property required for such metal sources is that they readily evaporate or sublime to give a metal containing vapor or gas which can be decomposed in a controlled manner to deposit a film onto a target substrate. Examples of such materials which are commonly utilized in the microelectronics industry in the preparation of printed circuits and semiconductor devices include the complex $H_3SiCo(CO)_4$ complex which is pyrolyzed in the gas phase at 670-770 °K to produce CoSi and dimethylzinc (1,4 dioxane) which is reacted with hydrogen selenide at 250-550 °C to produce ZnSe. References teaching the above CVD methods are B. J. Aylett, et al in Vacuum, 35 p 435-439(1985) and P. J. Wright, et al., in a paper accepted for publication in J. of Crystal Growth, London (1986), respectively.

Known fluorinated metal complexes that are chemically stable and easily volatized into the gas phase are the perfluorinated β-diketone metal coordination compounds along with their parent β-diketone precursor ligands, represented by the formulas:

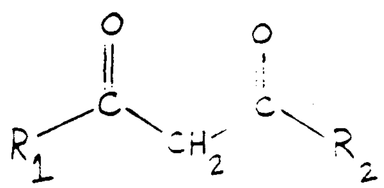

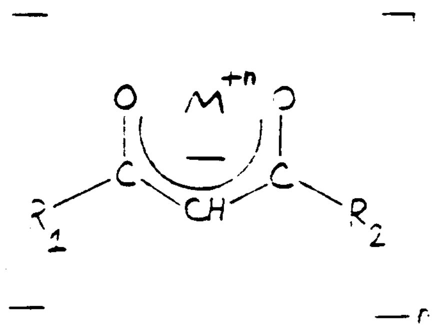

wherein $R_1$ is alkyl or fluoroalkyl, $R_2$ is fluoroalkyl, and $M^{+n}$ is a metal ion capable of forming a coordination compound. The volatility and gas phase stability of these compounds have been exploited for the gas chromatographic separation of various metals, the purification of uranium and the manufacture of specialty glasses. Decomposing such metal complexes by reaction with hydrogen in the gas phase to deposit thin metal films is taught in U.S. Patent 3,356,527.

In the past, attempts have been made to condense primary amines or primary diamines with ligands similar to those having the above structure. In instances in which $R_1$ and $R_2$ are not both fluorocarbon groups, it was reported that an O atom could be replaced with a N atom from an amine by direct Schiff-base condensation between an appropriate β-diketone and an amine. Additionally, the corresponding metal complex could be synthesized by chelation to a metal ion. See A. E. Martell, et al J. Inorg. Chem. Vol. 5 pp 170-181 (1958).

As reported by Sievers, et al in J. Inorg. Nucl. Chem. Vol. 32 pp 1895-1906 (1970), ligands in which $R_1$ and $R_2$ are both perfluoralkyl and in which an oxygen has been replaced with an amine have not been obtainable. It is believed that such methods have been unsuccessful because the perfluorinated β-diketones are of such high acidity that the amine used in the reaction becomes protonated, thereby forming a salt between the amine and the β-diketone rather than forming the desired ligand. Sievers, et al do report synthesizing a ligand having the structure:

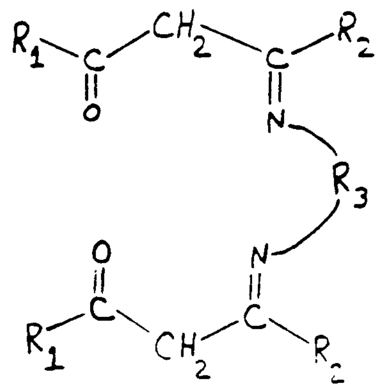

wherein $R_1 = R_2 = CF_3$ and $R_3 = -CH_2CH_2-$.

This ligand was reportedly synthesized by sublimation of the salt $[(CF_3C(O)CHC(O)CF_3)]_2^-$ $[NH_3\text{-}CH_2CH_2NH_3]^{+2}$. The ligand was reported to be chemically unstable and hence impossible to isolate.

Charles, U.S. Patent 3,594,216 discloses a process for depositing a metallic coating on a substrate by heating the substrate and contacting it with vaporized metal-organic beta-ketoamine chelates. The metal-organic beta-ketoamines were prepared by conventional synthesis techniques. While a wide range of metal chelates are disclosed generally, none of the examples or synthesis techniques specifically use perfluorinated metal chelates.

Johnson, et al in Journal of Fluorine Chemistry, <u>27</u> pp 371-378 (1985) reported synthesizing a ligand in which $R_1$ and $R_2$ are perfluoroalkyl and oxygen was replaced with an ammonia nitrogen. The $Cu^{+2}$ complex was also prepared and was reported to be volatile.

## BRIEF SUMMARY OF THE INVENTION

The present invention is a class of novel, β-ketoimine ligands and highly volatile metal complexes of the ligands and also a process for making the same. The β-ketoimine ligands of the present invention are those of the general structural formula:

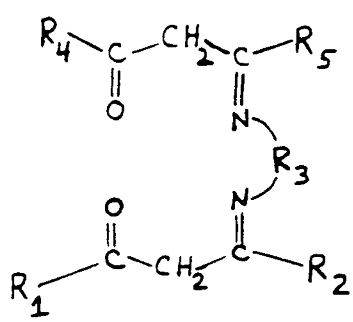

wherein $R_1$, $R_2$, $R_4$ and $R_5$ are independently linear or branched perfluorinated, $C_1$-$C_8$ alkyl groups and $R_3$ is any organic functionality such as a $C_1$-$C_8$ alkyl, phenyl or hydroxyalkyl group, all of which can be partially or fully fluorinated.

The highly volatile β-ketoiminato metal complexes which are synthesized from these ligands have the structural formula:

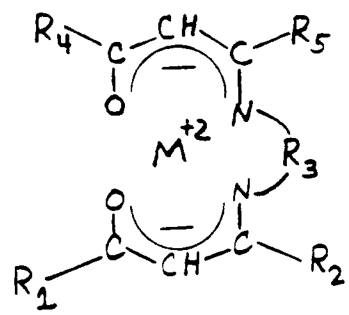

wherein $R_1$, $R_2$, $R_3$, $R_4$ and $R_5$ are as described above, and $M^{+2}$ is a divalent metal ion.

The present invention is also a process for making both the $\beta$-ketoimineligands and metal complexes described above. The ligands are synthesized by silylating a fluorinated $\beta$-diketone to form a silylenolether, and subsequently reacting the silylenolether with a primary diamine to form the desired ligand. The corresponding metal complex is formed by treating the resulting ligand with potassium methoxide followed by treatment with a halide salt of the desired metal.

## DETAILED DESCRIPTION OF THE INVENTION

The present invention is a class of heavily fluorinated $\beta$-ketoimine ligands and thermally volatilizable $\beta$-ketoiminato metal complexes of the ligands. The ligands are characterized in that they are highly fluorinated and contain oxygen and nitrogen donor atoms which can be covalently coordinated to a central metal atom to form the corresponding metal complex. This class of ligand, as well as the corresponding metal complex, are chemically stable and easily volatized into the gas phase. The high fluorine content of the complex is believed to reduce the Van der Waals forces between individual molecules and hence lower the boiling or sublimination point of the compound.

The heavily fluorinated $\beta$-ketoimine ligands of the present invention can be represented by the structural formula:

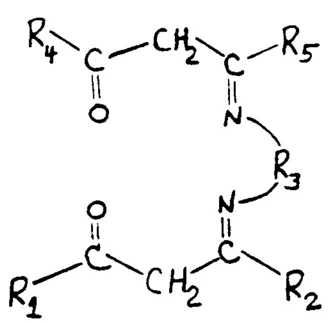

I

wherein $R_1$, $R_2$, $R_4$ and $R_5$ are independently linear or branched perfluorinated, $C_1$-$C_8$ alkyl groups and $R_3$ is any organic functionality such as a $C_1$-$C_8$ alkyl, phenyl or hydroxyalkyl group, all of which can be partially or fully fluorinated.

The highly volatile metal complexes which are synthesized from these ligands have the structural formula:

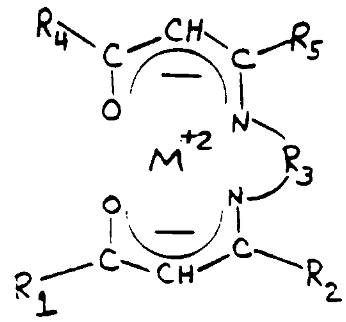

II

wherein $R_1$, $R_2$, $R_3$, $R_4$ and $R_5$ are as described above, and $M^{+2}$ is a is a divalent metal ion. These volatile complexes hold great potential for use as metal sources in Chemical Vapor Deposition (CVD) processes engaged in the art of depositing, for instance, metal films or metal oxide films.

The ligands of structure I above are synthesized by treating a fluorinated $\beta$-diketone of the formula $R_1COCH_2COR_2$ with potassium hydride under anhydrous conditions to produce a compound of the formula $R_1COCHCOR_2^- K^+$ and subsequently reacting the resultant $R_1COCHCOR_2^- K^+$ with a silylchloride such as, t-butyldimethylsilylchloride, to produce a silylenolether having the general formula:

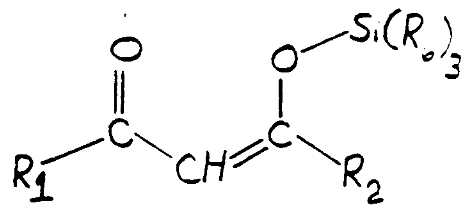

wherein $R_1$ and $R_2$ are as described above, and each $R_6$ is independently an alkyl group. The silylenolether described above is then treated with a primary diamine, $NH_2R_3NH_2$ wherein $R_3$ is as above to produce the desired $\beta$-ketoimine ligand of structural formula I.

To form the metal complex of the $\beta$-ketoimine ligand formed above, the ligand is initially treated with potassium methoxide and the resultant compound is subsequently treated with a metal halide of the formula $M^{+2}(X)_2^-$ , where X is a halogen, to form the desired highly fluorinated $\beta$-ketoiminato complex of formula II above.

The ligands of formula I produced in accordance with this invention can exist in two tautomeric forms, enol and keto, with the keto form being represented generally by formula I. Preferred ligands and metal complexes of the present invention include:

Ligands.

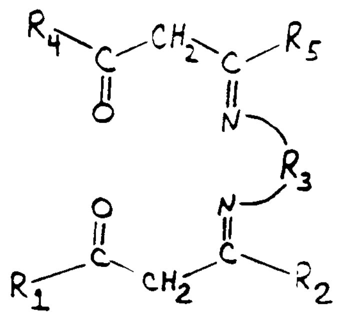

Where

$R_1 = R_2 = R_4 = R_5 = CF_3$, $R_3 = (CH_2)_2$

$R_1 = R_4 = CF_2CF_3$, $R_2 = R_5 = CF_3$, $R_3 = (CH_2)_2$

$R_1 = R_4 = CF_2CF_2CF_3$, $R_2 = R_5 = CF_3$, $R_3 = (CH_2)_2$

$R_1 = R_2 = R_4 = R_5 = CF_3$, $R_3 = (CH_2)_3$

$R_1 = R_2 = R_4 = R_5 = CF_3$, $R_3 = CH_2CH(OH)CH_2$

Complexes.

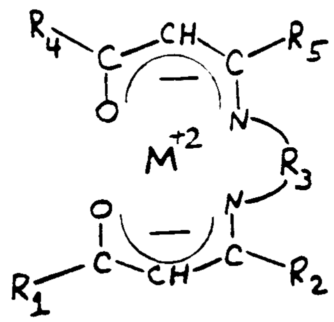

Where

$R_1 = R_2 = R_4\text{-}R_5 = CF_3$, $R_3 = (CH_2)_2$, $M^{+2} = Cu^{+2}$

$R_1 = R_2 = R_4\text{-}R_5 = CF_3$, $R_3 = (CH_2)_2$, $M^{+2} = Co^{+2}$

$R_1 = R_4 = CF_2CF_3$, $R_2 = R_5 = CF_3$, $R_3 = (CH_2)_2$ $M^{+2} = Cu^{+2}$

$R_1 = R_4 = CF_2CF_3$, $R_2 = R_5 = CF_3$, $R_3 = (CH_2)_2$ $M^{+2} = Co^{+2}$

$R_1 = R_4 = CF_2CF_2CF_3$, $R_2 = R_5 = CF_3$, $R_3 = (CH_2)_2$, $M^{+2} = Cu^{+2}$

$R_1 = R_4 = CF_2CF_2CF_3$, $R_2 = R_5 = CF_3$, $R_3 = (CH_2)_2$, $M^{+2} = Co^{+2}$

Experimental

In the following examples, temperatures are set forth uncorrected in degrees Celcius. Unless otherwise indicated, all parts and percentages are by weight.

1,1,1,5,5,5 hexafluoro-2,4-pentanedione, t-butyldimethylsilylchloride, potassium hydride, 2,2,2-trifluoroethylamine, ethylenediamine, ethanolamine, 1,3-propanediamine, 1,3-diamino-2-propanol and analine were obtained from Aldrich Chemical Co. (940 West St. Paul Ave. Milwaukee, Wis. 53233). 1,1,1,2,2,6,6,6-octafluoro-3,5-hexanedione and 1,1,1,2,2,3,3,7,7,7-decafluoro-4,6-heptanedione were obtained from Fairfield Chemical Company Inc. (P.O. Box 20, Blythewood, SC 29016).

Solvents used are HPLC grade. Tetrahydrofuran (THF) was distilled from calcium hydride under nitrogen, methanol was distilled from Mg metal under nitrogen. All operations in the preparation of the free ligands or corresponding complexes are carried out using Standard Schlenk line techniques described by

D. F. Shriver, "The Manipulation of Air Sensitive Compounds" McGraw-Hill Publishing Co.

Microanalyses were performed by Schwarzkopf Microanalytical Laboratory, Woodside, NY or Research Services, Air Products and Chemicals, Inc. $^1H$, $^{19}F$ and $^{13}C$ NMR spectra were recorded using an IBM SY-200 and a Bruker WH-200 NMR spectrometer.

The chemical structure, along with both the IUPAC and abbreviated names of the ligands synthesized in the following examples are set out below. The corresponding metal complexes have the similar structure with an (H) being replaced by the metal (see formula II above). The charge on the metal complex must remain neutral, i.e., if the ligand is diprotonated one divalent metal ion such as $Cu^{+2}$ is required.

1,2-di-[4-imino-1,1,1,5,5,5-hexafloro-2-pentanone] ethane
($H_2$)DODECA-F[EDA]

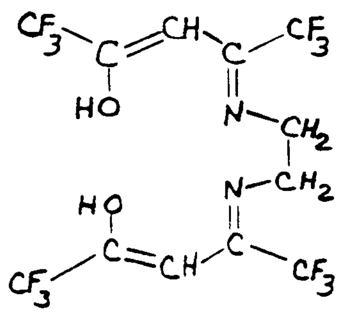

1,2-di-[5-imino-1,1,1,2,2,6,6-octafloro-3-hexanone] ethane
($H_2$)HEXADECA-F[EDA]

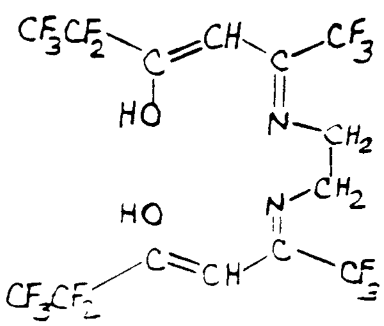

1,2-di-[6-imino-1,1,1,2,2,3,3,7,7,7-decafluoro-4-heptanone] ethane
($H_2$)EiCOSA-F[EDA]

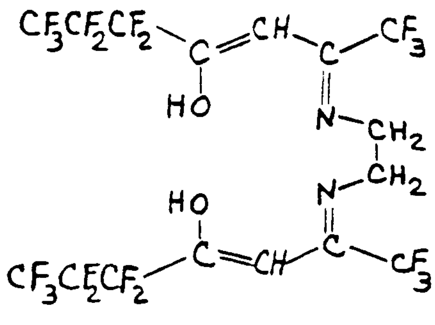

Bis[4(methylene)imino-1,1,1,5,5,5-hexafluoro-2-pentanone]methane
($H_2$)DODECA-F[PDA]

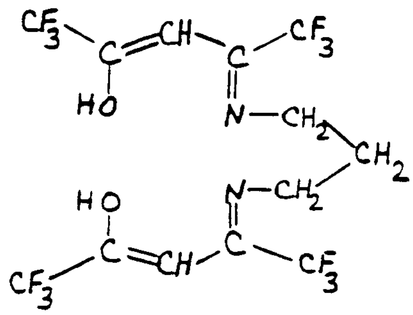

Bis[4(methylene)imino-1,1,1,5,5,5-hexafluoro-2-pentanone]methanol
($H_2$)DODECA-F[POA]

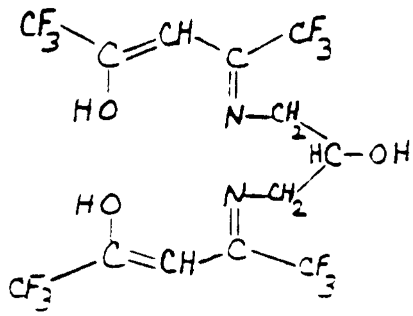

Example 1

Synthesis of the silylenolethers of perfluorinated β-diketones

The following represents a generic synthesis for the preparation of:

(i) 4-(t-butyldimethylsiloxy)-1,1,1,5,5,5-hexafluoro-3-penten-2-one from 1,1,1,5,5,5-hexafluoro-2,4-pentanediane.

(ii) 4-(T-butyldimethylsiloxy)-1,1,1,5,5,6,6,6 octafluoro-3-hexen-2-one (and its isomer 2-(t-butyldimethylisiloxy)-1,1,1,5,5,6,6,6-octafluoro-2-hexen-4-one) from 1,1,1,5,5,6,6,6-octafluoro-2,4-hexane dione.

(iii) 4-(t-butyldimethylsiloxy)-1,1,1,5,5,6,6,7,7,7-decafluoro-3-hepten-2-one (and its isomer 2-(t-butyldimethylsiloxy)-1,1,1,5,5,6,6,7,7,7-decafluoro-2-hepten-4-one) from 1,1,1,5,5,6,6,7,7,7-decafluoro-2-hepten-4-one.

Potassium hydride (20.0 g, 0.5 moles) is charged into a solid addition funnel which is fitted to a 1.01 reaction flask; the latter is also fitted with a rubber septum, an inlet for nitrogen, and a magnetic stir bar. Under an atmosphere of dry nitrogen THF (500 ml) is added to the flask which is subsequently cooled to -78°C. The perfluoro β-diketone (0.5 moles) is then added by syringe to the stirred THF at approx. 0.5 ml/min while also slowly adding potassium hydride at such a rate that it is consumed without an excess accumulating in the reaction flask. After adding all the reagents, the reaction is left to stir at room temperature until all traces of hydride are digested (up to 18 hrs). A reflux condenser and an addition funnel charged with t-butyldimethylsilylchloride (75.36 g. 0.5 moles) are fitted to the reaction flask, 150 ml THF is run into the addition funnel to dissolve the silylchloride. This solution is added dropwise over 30 mins to the stirring reaction mixture after which it is refluxed for 18 hrs. During this time a thick white precipitate of potassium chloride forms. The mixture is then filtered under nitrogen to give a pale brown or yellow filtrate. Approximately 500 ml of THF is then distilled off under nitrogen and the resulting concentrated silylenol ether solution left to cool, thereby precipitating further potassium chloride. This solution is then filtered as before then distilled under nitrogen to give the silylenolether as a moisture sensitive pale yellow liquid. NOTE: Care must be taken to not heat the distillation flask too near to dryness. As the residual brown colored liquid in the distillation flask evaporates down to ~30 ml it may start to evolve thick yellow-grey

fumes prior to rapidly decomposing and producing a surge of pressure within the apparatus.

It is noted that two silylenolether isomers form when starting with unsymmetrical perfluoro β-diketones (i.e., 1,1,1,2,2,6,6,6-octafluoro-3,5-hexadenione and 1,1,1,2,2,3,3,7,7,7-decafluoro-4,6-heptanedione. However, these are not separated in this distillation stage and are collected as one fraction composed of a mixture of two isomers. Each enolether is collected as one main fraction BPT 165-180 ° C.

The yields and analytical data are reported in Table 1.

TABLE 1

Yields and Analytical Data for Silylenol Ethers

| Starting β-Diketone | Silylenol Ether Formed | Isolated Yield | Boiling Point | NMR |
|---|---|---|---|---|
| 1,1,1,5,5,5-hexafluoro-2,4-pentane dione | 4-(t-butyldimethylsiloxy)-1,1,1,5,5,5-hexafluoro-3-penten-2-one | 64% | 165-175°C | 'H $CDCl_3$ δ0.32(S,6H);δ0.99(S,9H); δ6.27(S,1H)<br>$^{13}C$ $CDCl_3$ δ-4.34(S,2C);δ19.20(S,1C) δ25.51(S,3C);δ99.33(S,1C) δ116.0(Q,1C);δ120(Q,1C);δ156.1 (Q,1C);δ178(Q,1C)<br>$^{19}F$ $CDCl_3$ δ-76.5(S,3F);-70.2(S,3F) |
| 1,1,1,5,5,6,6,6-octafluoro-2,4-hexanedione | 4-(t-butyldimethylsiloxy)-1,1,1,5,5,6,6,6-octafluoro-3-hexen-2-one<br>and<br>2-(t-butyldimethylsiloxy)-1,1,1,5,5,6,6,6-octafluoro-2-hexen-4-one<br>(ie. two isomers) | 62%, | 165-175°C | 'H $CD_2Cl_2$ δ0.35(S,9H);δ1.0(S,6H); δ6.4(S,1H)<br>(only major isomer shown) |
| 1,1,1,5,5,6,6,7,7,7-decafluoro-2,4-heptanedione | 4-(t-butyldimethylsiloxy)-1,1,1,5,5,6,6,7,7,7-decafluoro-3-hepten-2-one<br>and<br>2-(t-butyldimethylsiloxy)-1,1,1,5,5,6,6,7,7,7-2-hepten-4-one<br>(i.e., two isomers) | 49%, | 165-180°C | 'H $CD_2Cl_2$ δ0.35(S,9H);δ1.0(S,6H); δ6.4(S,1H)<br>(only major isomer shown) |

Example 2

Preparation of ligands $(H_2)$DODECA-F[EDA], $(H_2)$HEXADECA-F[EDA], $(H_2)$EiCOSA-F[EDA], $(H_2)$DODECA-F-[PDA]

The above ligands are prepared by reaction of a diamine and a silylenolether of a perfluorinated $\beta$-diketone.

Under nitrogen a dry 100ml reaction flask fitted with a mechanical stirrer, addition funnel and rubber septum is charged with silylenolether (0.125 moles) and cooled to -78 °C. The addition funnel charged with diamine (0.0625 moles) dissolved in an equal volume of THF is added over 5 minutes to the stirring enolether. The mixture is then allowed to warm to room temperature where it is stirred for one further hour. During either the addition of the diamine or the warm up period a thick white precipitate of ligand forms. This solid is filtered off and washed with methanol, boiled down in fresh methanol until white crystals appear, then left to stand and cool. Filtration yields colorless needles. Yields of specific compounds are listed in Table 2.

TABLE 2

Analytical Data for Ligands (H2)DODECA-F[EDA], (H2)HEXADECA-F[EDA], (H2)EiCOSA-F[EDA], (H2)DODECA-F[PDA]

| Starting Enolether | Diamine | Ligand | Yield | MPt | Elemental Analysis | NMR |
|---|---|---|---|---|---|---|
| 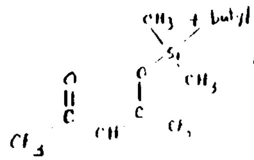 | $NH_2(CH_2)_2NH_2$ | (H2)DODECA-F[EDA] | 62% | 107°C | %calc for $C_{12}H_8F_{12}N_2O_2$: C 32.74; H 1.83; F 51.79; N 6.36; O 7.27<br>Found: C 32.79; H 1.92; F 51.72; N 6.36 | $^1H$ $CDCl_3$ δ3.77(m,4H);δ5.87(S,2H); δ10.55(bs,2H)<br>$^{19}F$ $CDCl_3$ δ-66.2(S);δ-77.0(S)<br>$^{13}C$ $CDCl_3$ δ46.28;δ6.76;δ117.54; δ120.17;δ154.56;δ179.78 |
| 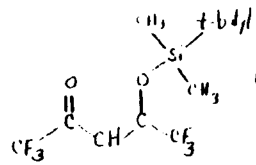 | $NH_2(CH_2)_3NH_2$ | (H2)DODECA-F[PDA] | 53% | 70-75°C | %calc for $C_{13}H_{10}N_2O_2F_{12}$: C 34.38; H 2.22; N 6.17; O 7.05; F 50.19<br>Found: C 34.07; H 2.11; N 6.53; O ; F 45.6 | $^1H$ $CDCl_3$ δ2.07(p,2H);δ3.6(Q,4H); δ5.87(S,2H);δ10.5(bs,2H)<br>$^{19}F$ $CDCl_3$ δ-77.8(S);δ-67.4(S)<br>$^{13}C$ $CDCl_3$ δ-30.75(S);δ36.89(S) δ86.56(S);δ116.15(Q);δ119.0(Q); δ154.04(Q); δ180.07(Q) |
| 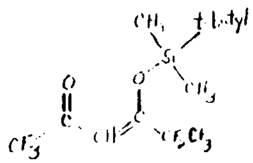 | $NH_2(CH_2)_2NH_2$ | (H2)HEXADECA-F[EDA] | 42% | 77-79°C | %calc for $C_{14}H_8N_2O_2F_{16}$: C 31.13; H 1.49; N 5.19; O 5.92; F 56.27<br>Found: C 30.80; H 1.45; N 6.11; O ; F 54.3 | $^1H$ $CDCl_3$ δ3.75(t,2H);δ6.0(S,2H); δ10.6(bs,2H)<br>$^{19}F$ $CDCl_3$ δ-123.97(S);δ-82.81(S); δ-66.89(S)<br>$^{13}C$ $C_6D_6$ δ44.18(S);δ88.30(S); δ108(tQ);δ118.88(Qt);δ119.13(Q), δ153.21(Q);δ182.26(t) |
| 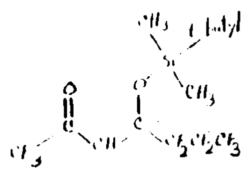 | $NH_2(CH_2)_2NH_2$ | (H2)EiCOSA-F[EDA] | 33% | 70-74°C | %calc for $C_{16}H_8N_2O_2F_{20}$: C 30.02; H 1.25; N 4.38; O 5.00; F 59.35<br>Found: C 29.63; H 1.16; N 5.00; O ; F 53.3 | $^1H$ $CD_3COCD_3$ δ4.1(S,4H);δ5.9(S,2H); δ10.7(bs,2H)<br>$^{19}F$ $CD_3COCD_3$ δ-65.39(S); δ-80.15(t);δ-120.36(d); δ-126.31(S)<br>$^{13}C$ $CD_3COCD_3$ δ46.36(S);δ88.08(S); δ109.97(tm);δ109.97(tt);δ112.94 (Qt);δ120.22(Q);δ154.49(Q) |

Example 3

Preparation of ligand ($H_2$)DODECA-F[POA]

Under a cover of nitrogen, a 100 ml reaction flask fitted with a reflux condenser, addition funnel and magnetic stir bar is charged with the t-butyl dimethylsilylenolether of hexafluoro-2,4-pentadione (9.66 g, 3 x $10^{-2}$ moles). The addition funnel is charged with 1,3-diamino-2-propanol (POA) (1.35g, 1.5 X $10^{-2}$ moles) in 5ml THF and this solution is then added over 5 mins, with stirring, to the enolether after which the reaction mixture is refluxed 15 hrs.

Upon cooling and standing overnight a buff colored solid forms which is filtered off and recrystallized from hexane/$CH_2Cl_2$ 50/50 to give colorless blocks. The yield was 6.5%. Analytical data is reported in Table 3 below.

TABLE 3

Analytical Data for (H2)DODECA-F[POA]

| Starting Enolether | Alkanolamine | Ligand Formed | NMR |
|---|---|---|---|
| 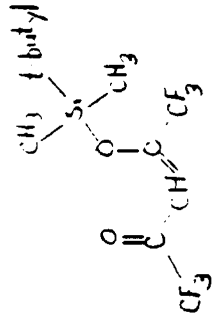 | 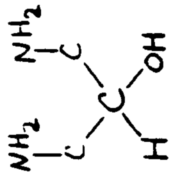 | (H2)DODECA-F[POA] | ¹H CDCl₃ δ2.3(bs,1H);δ3.6(m,4H);<br>δ4.15(m,1H);δ5.9(S,2H);<br>δ10.7(bs,2H)<br>$^{19}$F CDCl₃ δ-77.8(S);δ-67.4(S)<br>$^{13}$C CDCl₃ δ47.97(S);δ68.04(S);<br>δ100.12(S);δ116.5(Q);δ118.93(Q);<br>δ154.04(Q);δ180.0(Q) |

Example 4

Preparation of metal complexes of Perfluoro bis-(β-ketoimine) ligands

Under nitrogen, potassium methoxide (1.75 g, 0.025 moles) is dissolved in 150 ml dry methanol and 0.0125 mole of a bis-(β-ketoimine) ligand is added. The mixture is then stirred for 15 minutes to give a clear

bright yellow solution. Solid metal dibromide (0.0125 moles) is then added and the mixture stirred an additional 1 hour. The mixture is then filtered, the methanol evaporated off from the filtrate and the resultant solid redissolved in toluene (100 ml). This solution is filtered to remove residual potassium bromide and the filtrate evaporated to a solid that is then sublimed under a dynamic vacuum to yield the product complex. The analytical data is reported in Table 4 below:

TABLE 4

Analytical Data for perfluoro ß-iminoketone and Bis-(perfluoro ß-iminoketone) Metal Complexes

| Ligand | Metal Bromide | Complex | Yield | MPt | Mass Spectra | Elemental Analysis |
|---|---|---|---|---|---|---|
| (H2)DODECA-F[EDA] | $CoBr_2$ | $Co^{+2}$DODECA-F[EDA] | 74.2% | 111°C | Cal 496.9570<br>Found 496.9533 | Calculated for: $C_{12}H_6N_2O_2F_{12}Co$<br>% C 28.99; H 1.22; N 5.63; O 6.44; F 45.86; Co 11.85<br>Found C 29.26; H 1.32; N 4.15; O ; F 41.40; Co 10.60 |
| (H2)DODECA-F[EDA] | $CuBr_2$ | $Cu^{+2}$DODECA-F[EDA] | 92.3% | 132-135°C | Cal 500.9534<br>Found 500.9512 | Calculated for: $C_{12}H_6N_2O_2F_{12}Cu$<br>% C 28.73; H 1.21; N 5.58; O 6.38; F 45.44; Cu 12.67<br>Found C 28.85; H 1.20; N 4.03; O ; F 40.10; Cu 12.30 |
| (H2)HEXADECA-F[EDA] | $CoBr_2$ | $Co^{+2}$HEXADECA-F[EDA] | 67% | 75-80°C | Cal 596.9506<br>Found 596.9511 | Calculated for: $C_{14}H_6N_2O_2F_{16}Co$<br>% C 28.16; H 1.01; N 4.69; O 5.36; F 50.91; Co 9.87<br>Found C 27.52; H 1.07; N 3.80; O ; F 46.20; Co 9.53 |
| (H2)HEXADECA-F[EDA] | $CuBr_2$ | $Cu^{+2}$HEXADECA-F[EDA] | 87.6% | 112-120°C | Cal 600.9470<br>Found 600.9467 | Calculated for: $C_{14}H_6N_2O_2F_{16}Cu$<br>% C 27.94; H 1.01; N 4.65; O 5.32; F 50.52; Cu 10.56<br>Found C 28.25; H 0.88; N 3.98; O ; F 48.20; Cu 10.20 |
| (H2)EiCOSA-F[EDA] | $CoBr_2$ | $Co^{+2}$EiCOSA-F[EDA] | 91.6% | 82-84°C | Cal 696.9442<br>Found 696.9450 | Calculated for: $C_{16}H_6N_2O_2F_{20}Co$<br>% C 27.57; H 0.87; N 4.02; O 4.59; F 54.50; Co 8.4[illegible]<br>Found C 27.49; H 0.76; N 3.82; O ; F 49.50; Co 6.09 |
| (H2)EiCOSA-F[EDA] | $CuBr_2$ | $Cu^{+2}$EiCOSA-F[EDA] | 90.1% | 99-102°C | Cal 700.9406<br>Found 700.9437 | Calculated for: $C_{16}H_6N_2O_2F_{20}Cu$<br>% C 27.39; H 0.86; N 3.99; O 4.56; F 54.15; Cu 9.0[illegible]<br>Found C 27.62; H 0.72; N 3.67; O ; F 55.40; Cu 8.70 |

Example 5

Relative volatility measurement of perfluoro β-ketoiminato complexes

A standard weight loss experiment for each metal complex was conducted by heating a sample of approximately 50 mg at 10 °C/min under a 100 cc/min flow of nitrogen using a DuPont Model No. 951. Thermal Gravimetric Analyzer in conjunction with a model No. 9900 Controller. Table 5 below indicates that all of the metal complexes tested in this way are clearly volatile, leaving as little as 0.641% residue at the end of an evaporation cycle. The data reported indicates that the evaporation of these complexes is a smooth process, i.e. a gradual and even transition from the solid to the gas phase.

TABLE 5

| Volatility of Perfluoro bis-(β-ketoiminato) Copper Complexes | | |
|---|---|---|
| Complex | Temperature T °C at which complex is completely vaporized | Residual weight left at T °C |
| $Cu^{+2}$DODECA-F[EDA] | 245 | 1.07% |
| $Cu^{+2}$HEXADECA-F[EDA] | 245 | 1.94% |
| $Cu^{+2}$EICOSA-F[EDA] | 245 | 0. 641% |

Having thus described the present invention, what is now deemed appropriate for Letters Patent in set out in the following appended claims.

**Claims**

**1.** A chemically stable, β-ketoimine ligand having the structural formula:

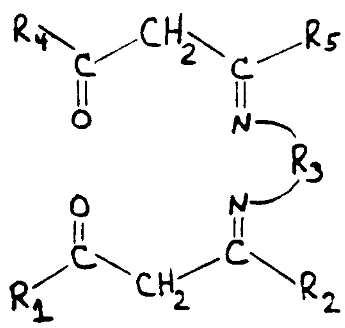

wherein $R_1$, $R_2$, $R_4$ and $R_5$ are independently linear or branched, perfluorinated $C_1$-$C_8$ alkyl groups, and $R_3$ is an organic functionality.

**2.** A ligand in accordance with Claim 1 wherein $R_3$ is a $C_1$-$C_8$ alkyl, phenyl or hydroxyalkyl group.

**3.** A ligand in accordance with Claim 1 wherein $R_3$ is a $C_1$-$C_8$ alkyl, phenyl or hydroxyalkyl group which is at least partially fluorinated.

**4.** A ligand in accordance with Claim 1 wherein $R_1$, $R_2$, $R_4$ and $R_5$ are $CF_3$.

**5.** A ligand in accordance with Claim 1 wherein $R_1$ and $R_2$ are independently perfluorinated methyl, ethyl or propyl groups.

**6.** A ligand in accordance with Claim 5 wherein $R_3$ is $CH_2CH_2$.

7. A ligand in accordance with Claim 1 wherein said ligand is 1,2-di-[4-imino-1,1,1,5,5,5-hexafluoro-2-pentanone]ethane.

8. A ligand in accordance with Claim 1 wherein said ligand is 1,2-di[5-imino-1,1,1,2,2,6,6,6-octafluoro-3-hexanone]ethane.

9. A ligand in accordance with Claim 1 wherein said ligand is 1,2-di-[6-imino-1,1,1,2,2,3,3,7,7,7-decafluoro-4-heptanone]ethane.

10. A thermally volatilizable, $\beta$-ketoiminato metal complex having the structural formula:

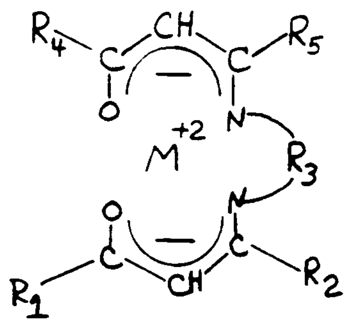

wherein $R_1$, $R_2$, $R_4$ and $R_5$ are independently linear or branched, perfluorinated, $C_1$-$C_8$ alkyl groups, $R_3$ is an organic functionality, $M^{+2}$ is a divalent metal ion.

11. A metal complex in accordance with Claim 10 wherein $R_3$ is a $C_1$-$C_8$ alkyl, phenyl or hydroxyalkyl group.

12. A metal complex in accordance with Claim 10 wherein $R_3$ is a $C_1$-$C_8$ alkyl, phenyl or hydroxyalkyl group which is at least partially fluorinated.

13. A metal complex in accordance with Claim 10 wherein $R_1$, $R_2$, $R_4$ and $R_5$ are $CF_3$.

14. A metal complex in accordance with Claim 10 wherein $R_1$ and $R_2$ are independently perfluorinated methyl, ethyl or propyl groups.

15. A metal complex in accordance with Claim 14 wherein $R_3$ is $CH_2CH_2$.

16. A metal complex in accordance with Claim 10 wherein $M^{+2}$ is $Cu^{+2}$.

17. A metal complex in accordance with Claim 10 wherein $M^{+2}$ is $Co^{+2}$.

**18.** A process for making a stable, β-ketoimine ligand having the structural formula:

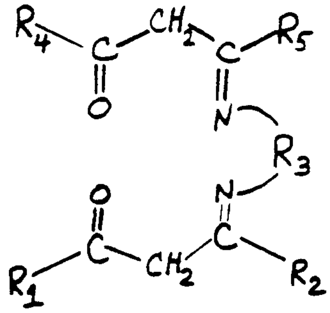

wherein $R_1$, $R_2$, $R_4$ and $R_5$ are independently linear or branched, perfluorinated $C_1$-$C_8$ alkyl groups, and $R_3$ is an organic functionality, said process comprising:

a) treating β-diketones of the formulae $R_1COCH_2COR_2$ and $R_4COCH_2COR_5$ with potassium hydride under anhydrous conditions to produce compounds of the formulae $R_1COCHCOR_2^- K^+$ and $R_4COCHCOR_5^- {}^{K+}$;

b) treating the resultant $R_1COCHCOR_2^- K^+$ and $R_4COCHCOR_5^- {}^{K+}$ with a silylchloride of the formula $(R_6)_3SiCl$, wherein each $R_6$ is independently an alkyl group, to produce silylenolethers of the formulae

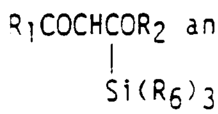

and

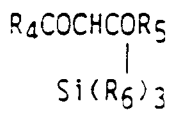

wherein

$R_1$, $R_2$, $R_4$, $R_5$, and $R_6$ are as above; and

(c) treating said silylenolethers with a primary diamine of the formula $NH_2R_3NH_2$ wherein $R_3$ is as described above to produce the desired B-ketoimine ligand.

**19.** A process in accordance with Claim 18 wherein said silylchloride is t-butyldimethylsilylchloride.

**20.** A process in accordance with Claim 18 wherein $R_3$ is a $C_1$-$C_8$ alkyl, phenyl or hydroxyalkyl group.

**21.** A process in accordance with Claim 18, wherein the desired β-ketoimine ligand produced in step (c) is treated with potassium methoxide and the resultant compound is subsequently treated with a metal halide of the formula $M^{+2}(X)_2^-$, wherein X is a halogen, to form a β-ketoiminato metal complex.

**Patentansprüche**

1. Chemisch stabiler β-Ketoimin-Ligand mit der Strukturformel

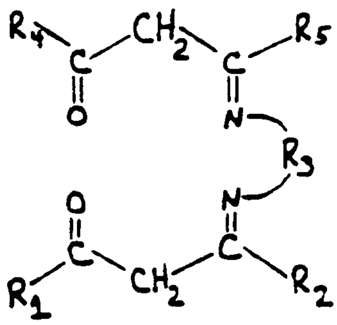

worin $R_1$, $R_2$, $R_4$ und $R_5$ unabhängig voneinander lineare oder verzweigte perfluorierte $C_1$-$C_8$-Alkylgruppen sind und $R_3$ eine organische Funktionalität ist.

2. Ligand nach Anspruch 1, wobei $R_3$ eine $C_1$-$C_8$-Alkyl-, Phenyl- oder Hydroxyalkylgruppe ist.

3. Ligand nach Anspruch 1, wobei $R_3$ eine $C_1$-$C_8$-Alkyl-, Phenyl- oder Hydroxyalkylgruppe ist, die zumindest teilfluoriert ist.

4. Ligand nach Anspruch 1, wobei $R_1$, $R_2$, $R_4$ und $R_5$ $CF_3$ sind.

5. Ligand nach Anspruch 1, wobei $R_1$ und $R_2$ unabhängig voneinander perfluorierte Methyl-, Ethyl- oder Propylgruppen sind.

6. Ligand nach Anspruch 5, wobei $R_3$ $CH_2CH_2$ ist.

7. Ligand nach Anspruch 1, wobei der Ligand 1,2-Di-[4-imino-1,1,1,5,5,5-hexafluor-2-pentanon]ethan ist.

8. Ligand nach Anspruch 1, wobei der Ligand 1,2-Di-[5-imino-1,1,1,2,2,6,6,6-octafluor-2-hexanon]ethan ist.

9. Ligand nach Anspruch 1, wobei der Ligand 1,2-Di-[6-imino-1,1,1,2,2,3,3,7,7,7-decafluor-4-heptanon]-ethan ist.

10. β-Ketoiminato-Metallkomplex, der durch Wärme verflüchtigt werden kann, mit der Strukturformel

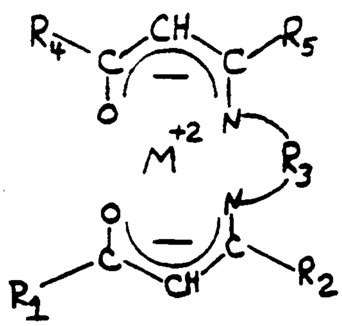

worin $R_1$, $R_2$, $R_4$ und $R_5$ unabhängig voneinander lineare oder verzweigte perfluorierte $C_1$-$C_8$-Alkylgruppen sind und $R_3$ eine organische Funktionalität ist, $M^{+2}$ ein zweiwertiges Metallion ist.

11. Metallkomplex nach Anspruch 10, wobei $R_3$ eine $C_1$-$C_8$-Alkyl-, Phenyl- oder Hydroxyalkylgruppe ist.

**12.** Metallkomplex nach Anspruch 10, wobei $R_3$ eine $C_1$-$C_8$-Alkyl-, Phenyl- oder Hydroxyalkylgruppe ist, die zumindest teilfluoriert ist.

**13.** Metallkomplex nach Anspruch 10, wobei $R_1$, $R_2$, $R_4$ und $R_5$ $CF_3$ sind.

**14.** Metallkomplex nach Anspruch 10, wobei $R_1$ und $R_2$ unabhängigvoneinander perfluorierte Methyl-, Ethyl- oder Propylgruppen sind.

**15.** Metallkomplex nach Anspruch 14, wobei $R_3$ $CH_2CH_2$ ist.

**16.** Metallkomplex nach Anspruch 10, wobei $M^{+2}$ $Cu^{+2}$ ist.

**17.** Metallkomplex nach Anspruch 10, wobei $M^{+2}$ $Co^{+2}$ ist.

**18.** Verfahren zur Herstellung eines stabilen $\beta$-Ketoimin-Liganden der Strukturformel:

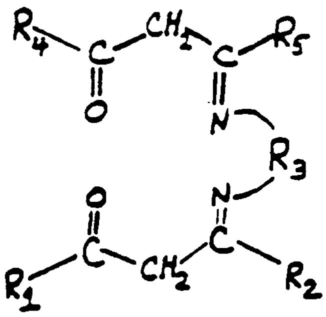

worin $R_1$, $R_2$, $R_4$ und $R_5$ unabhängig voneinander lineare oder verzweigte perfluorierte $C_1$-$C_8$-Alkylgruppen sind und $R_3$ eine organische Funktionalität ist, wobei das Verfahren umfaßt:

a) Behandeln von $\beta$-Diketonen der Formel $R_1COCH_2COR_2$ und $R_4COCH_2COR_5$ mit Kaliumhydrid bei wasserfreien Bedingungen, wodurch Verbindungen der Formel $R_1COCHCOR_2^-K^+$ und $R_4COCHCOR_5^-K^+$ hergestellt werden;

b) Behandeln des entstandenen $R_1COCHCOR_2^-K^+$ und $R_4COCHCOR_5^-K^+$ mit Silylchlorid der Formel $(R_6)_3SiCl$, wobei jedes $R_6$ unabhängig eine Alkylgruppe ist, wodurch Silylenolether der Formel

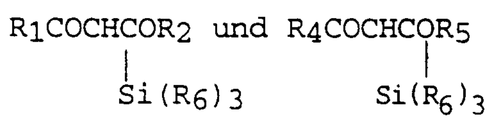

hergestellt werden. wobei

$R_1$, $R_2$, $R_4$, $R_5$ und $R_6$ wie oben sind, und

c) Behandeln der Silylenolether mit einem primären Diamin der Formel $NH_2R_3NH_2$, wobei $R_3$ wie oben beschrieben ist, wodurch der gewünschte $\beta$-Ketoimin-Ligand hergestellt wird.

**19.** Verfahren nach Anspruch 18, wobei das Silylchlorid t-Butyldimethylsilylchlorid ist.

**20.** Verfahren nach Anspurch 18, wobei $R_3$ eine $C_1$-$C_8$-Alkyl-, Phenyl- oder Hydroxyalkylgruppe ist.

**21.** Verfahren nach Anspruch 18, wobei der im Schritt (c) hergestellte gewünschte $\beta$-Ketoimin-Ligand mit Kaliummethoxid behandelt und die entstandene Verbindung anschließend mit einem Metallhalogenid der Formel $M^{+2}(X)_2^-$ behandelt wird, wobei X ein Halogenatom ist, wodurch der $\beta$-Ketoiminato-Metallkomplex gebildet wird.

**Revendications**

1. Ligand de $\beta$-cétoimine chimiquement stable ayant la formule de structure :

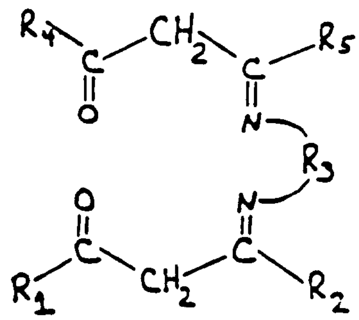

dans laquelle $R_1$, $R_2$, $R_4$ et $R_5$ sont indépendamment des groupes alkyle linéaires ou ramifiés perfluorés $C_1$-$C_8$ et $R_3$ est une fonctionnalité organique.

2. Ligand selon la revendication 1, dans lequel $R_3$ est un groupe alkyle, phényle ou hydroxyalkyle en $C_1$-$C_8$.

3. Ligand selon la revendication 1, dans lequel $R_3$ est un groupe alkyle, phényle ou hydroxyalkyle en $C_1$-$C_8$ qui est au moins partiellement fluoré.

4. Ligand selon la revendication 1, dans lequel $R_1$, $R_2$, $R_4$ et $R_5$ sont $CF_3$.

5. Ligand selon la revendication 1, dans lequel $R_1$ et $R_2$ sont des groupes méthyle, éthyle ou propyle indépendamment perfluorés.

6. Ligand selon la revendication 5, dans lequel $R_3$ est $CH_2CH_2$.

7. Ligand selon la revendication 1, dans lequel le ligand est un 1,2-di-[4-imino-1,1,1,5,5,5-hexafluoro-2-pentanone] éthane.

8. Ligand selon la revendication 1, dans lequel le ligand est 1,2-di[5-imino-1,1,1,2,2,6,6,6-octafluoro-3-hexanone] éthane.

9. Ligand selon la revendication 1, dans lequel le ligand est 1,2-di-[6-imino-1,1,1,2,2,3,3,7,7,7-decafluoro-4-heptanone]éthane.

10. Complexe métallique $\beta$-cétoiminato thermiquement volatilisable ayant la formule de structure :

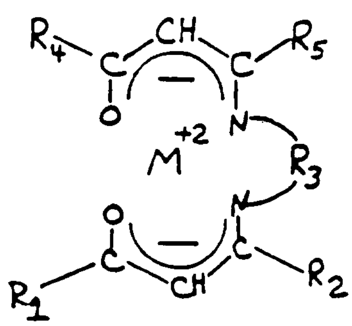

dans laquelle $R_1$, $R_2$, $R_4$ et $R_5$ sont des groupes alkyle linéaires ou ramifiés perfluorés $C_1$-$C_8$, $R_3$ est une fonctionnalité organique $M^{+2}$ est un ion métallique divalent.

**11.** Complexe métallique selon la revendication 10, dans lequel $R_3$ est un groupe alkyle, phényle ou hydroxyalkyle $C_1$-$C_8$.

**12.** Complexe métallique selon la revendication 10, dans lequel $R_3$ est un groupe alkyle, phényle ou hydroxyalkyle $C_1$-$C_8$ qui est au moins partiellement fluoré.

**13.** Complexe métallique selon la revendication 10, dans lequel $R_1$, $R_2$, $R_4$ et $R_5$ sont $CF_3$.

**14.** Complexe métallique selon la revendication 10, dans lequel $R_1$ et $R_2$ sont des groupes méthyle, éthyle ou propyle indépendamment perfluorés.

**15.** Complexe métallique selon la revendication 14, dans lequel $R_3$ est $CH_2CH_2$.

**16.** Complexe métallique selon la revendication 10, dans lequel $M^{+2}$ est $CU^{+2}$.

**17.** Complexe métallique selon la revendication 10, dans lequel $M^{+2}$ est $Co^{+2}$.

**18.** Procédé pour réaliser un ligand $\beta$-cétoimine stable ayant la formule de structure :

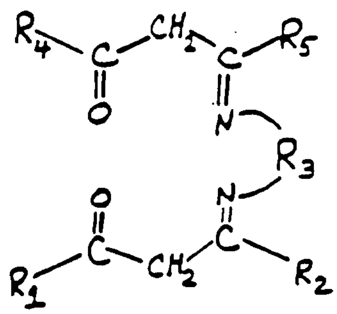

dans laquelle $R_1$, $R_2$, $R_4$ et $R_5$ sont indépendamment des groupes alkyle en $C_1$-$C_8$ linéaires ou ramifiés perfluorés et $R_3$ est une fonctionnalité organique, le procédé comprenant :

a) Traitement des $\beta$-dicétones des formules $R_1COCH_2COR_2$ et $R_4COCH_2COR_5$ avec de l'hydrure de potassium dans des conditions anhydres pour produire des composés des formules $R_1COCHCOR^{-}{}_2K^+$ et $R_4COCHCOR^{-}{}_5{}^{K+}$;

b) traitement du $R_1COCHCOR^{-}{}_2K+$ et $R_4COCHCOR^{-}{}_5{}^{K+}$ résultant avec du chlorure de silyle de la formule $(R_6)_3SiCl$, dans laquelle chaque $R_6$ est indépendamment un groupe alkyle pour produire des silylénoléthers des formules

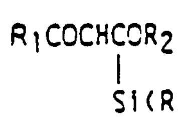 and 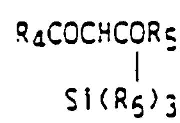

dans lesquelles $R_1$, $R_2$, $R_4$, $R_5$ et $R_6$ sont telles que ci-dessus; et

c) traitement des silylénoléthers avec une diamine primaire de la formule $NH_2R_3NH_2$ dans laquelle $R_3$ est tel que décrit ci-dessus pour produire le ligand $\beta$-cétoimine souhaité.

**19.** Procédé selon la revendication 18, dans lequel le chlorure de silyle est du chlorure de t-butyldiméthylsilyle.

**20.** Procédé selon la revendication 18, dans lequel $R_3$ est un groupe alkyle, phényle ou hydroxyalkyle $C_1$-$C_8$.

**21.** Procédé selon la revendication 18, dans lequel le ligand $\beta$-cétoimine souhaité produit à l'état (c) est traité avec du méthoxide de potassium et le composé résultant est ensuite traité avec un halogénure métallique de la formule $M^{+2}(X)^{-}_{2}$, dans laquelle X est un halogène pour former un complexe métallique $\beta$-cétoiminato.